# EUROPEAN PATENT SPECIFICATION

(11) **EP 1 227 804 B1**
(45) Date of publication and mention of the grant of the patent: **22.08.2007**
(21) Application number: 00977121.3
(22) Date of filing: 08.11.2000
(51) Int. Cl.: A61K 31/585, A61L 31/16, A61P 9/00

(54) **USE OF EPLERENONE FOR TREATING RESTENOSIS**
VERWENDUNG VON EPLERENON ZUR BEHANDLUNG VON RESTENOSE
UTILISATION DE L'EPLERENONE POUR LE TRAITEMENT DE LA RESTENOSE

(30) Priority: 09.11.1999 US 164390 P
(43) Date of publication of application: 07.08.2002
(73) Proprietor: Pharmacia Corporation, Peapack, NJ 07977 (US)
(72) Inventor: DELYANI, John, A., Annandale, NJ 08801 (US); FEDDE, Kenton, N., Webster Groves, MO 63119 (US); FUNDER, John, W., Prahan, VIC (AU); WARD, Michael, R., St. Leonards, NSW 2065 (AU); KANELLAKIS, Peter, Melbourne, Victoria 8008 (AU); BOBIK, Alex, Melbourne, Victoria 8008 (AU)
(74) Representative: Albrecht, Thomas
(86) International application number: PCT/US2000/030853
(87) International publication number: WO 2001/034132

(56) References cited:
- WO-A-00/10552
- WO-A-95/15166
- WO-A-96/40255
- WARD M.R. ET AL: "Eplerenone suppresses constrictive remodeling and collagen accumulation after angioplasty in porcine coronary arteries." CIRCULATION, (24 JUL 2001) 104/4 (467-472). , XP001027832
- VAN BELLE E ET AL: "Neointimal thickening after balloon denudation is enhanced by aldosterone and inhibited by spironolactone, and aldosterone antagonist." CARDIOVASCULAR RESEARCH, (1995 JAN) 29 (1) 27-32. , XP001025753 cited in the application
- VAN BELLE, E. (1) ET AL: "Aldosterone: A potent modulator of the vascular response to injury." EUROPEAN HEART JOURNAL, (1994) VOL. 15, NO. ABSTR. SUPPL., PP. 116. MEETING INFO.: JOINT XIITH WORLD CONGRESS OF CARDIOLOGY AND THE XVITH CONGRESS OF THE EUROPEAN SOCIETY OF CARDIOLOGY BERLIN, GERMANY SEPTEMBER 10-14, 1994 , XP001027890
- KLAUBER, NANCY ET AL: "New activity of spironolactone: Inhibition of angiogenesis in vitro and i vivo." CIRCULATION, (1996) VOL. 94, NO. 10, PP. 2566-2571. , XP001025755 cited in the application
- EPSTEIN M ET AL: "EPLERENONE A NEW SELECTIVE ALDOSTERONE RECEPTROR ANTAGONIST (SARA): EFFICACY IN PATIENTS WITH MILD TO MODERATE HYPERTENSION" JOURNAL OF THE AMERICAN SOCIETY OF NEPHROLOGY, WILLIAMS AND WILKINS, BALTIMORE, MD, US, vol. 9, September 1998 (1998-09), pages 322A-323A, XP001003077 ISSN: 1046-6673
- EPSTEIN M ET AL: "EPLERENONE, A NOVEL AND SELECTIVE ALDOSTERONE RECEPTOR ANTAGONIST: EFFICACY IN PATIENTS WITH MILD TO MODERATE HYPERTENSION" CIRCULATION, AMERICAN HEART ASSOCIATION, DALLAS, TX, US, vol. 98, no. 17, SUPPL, 27 October 1998 (1998-10-27), pages I98-I99, XP001001544 ISSN: 0009-7322
- RABASSEDA X ET AL: "ANTIHYPERTENSIVE TREATMENT OF HEART FAILURE ALDOSTERONE ANTAGONIST" DRUGS OF THE FUTURE, BARCELONA, ES, vol. 24, no. 5, 1999, pages 488-501, XP001001569 ISSN: 0377-8282
- SLIGHT, SIMON H. (1) ET AL: "Extra-adrenal mineralocorticoids and cardiovascular tissue." JOURNAL OF MOLECULAR AND CELLULAR CARDIOLOGY, (JUNE, 1999) VOL. 31, NO. 6 PP. 1175-1184. , XP001025711
- BENETOS A ET AL: "Prevention of aortic fibrosis by spironolactone in spontaneously hypertensive rats." ARTERIOSCLEROSIS, THROMBOSIS, AND VASCULAR BIOLOGY, (1997 JUN) 17 (6) 1152-6. , XP001025767
- ROCHA, RICARDO ET AL: "Mineralocorticoid blockade reduces vascular injury in stroke-prone hypertensive rats" HYPERTENSION (1998), 31(1, PT. 2), 451-458 , XP001027834

## Description

### BACKGROUND OF THE INVENTION

### Field of the Invention

This invention relates generally to methods (not claimed as such) for treating, inhibiting or preventing restenosis in a human subject who has undergone angioplasty by administering a therapeutically-effective amount of eplerenone.

### Description of the Related Art

Vascular injury can result from a variety of causes including elective and emergency surgical events, trauma, aneurism, ischemia, infarction and the like Medical procedures such as percutaneous transluminal coronary angioplasty ("PTCA") in the treatment of patients with atherosclerotic coronary disease commonly result in vascular injury. Arterial injury due to PTCA triggers healing processes that can further lead to pathogenic events that cause constrictive remodeling of the artery. Constrictive remodeling is considered to be a major contributor to restenosis after PTCA and results in lumenal narrowing and a reduction in blood flow through the area encompassed by the external elastic lamina. Mintz et al., Circulation, Vol. 94, pp. 35-43 (1996). Other studies using porcine models of PTCA have confirmed this observation and report that late luminal loss substantially exceeds that luminal loss attributed to neointimal formation associated with the normal healing process. Andersen, et al., Circulation, Vol. 93, pp. 1716-1724 (1996).

Restenosis generally results from a complex post-injury sequence of events characterized by neointimal hyperplasia and involving monocytes, macrophages and smooth muscle cell activation and migration into subintima. In response to various cytokines, smooth muscle cells proliferate and elaborate excessive connective tissue and extracellular matrix. In the clinical arena, this problem has been attenuated by using endolumenal stents which mechanically oppose this constrictive process or drug therapies such as administration of heparin. Restenosis, however, still occurs despite the stent or drug therapy, primarily by a process of excessive neointimal growth during vessel healing. Serruys et al., N. Engl. J. Med., Vol. 331, pp. 489-495 (1994)..

The role of smooth muscle cells in restenosis has been reported in animal models. Clowes et al., J. Cardiovasc. Pharmacol., Vol. 14 (Supp. 6), pp. S 12-15 (1989). Similarly, the role of smooth muscle cells in restenosis has been reported in man. Austin et al., J. Am. Coll. Cardiol. Vol. 6, pp. 369-75 (1985). The smooth muscle cells secrete collagen which is a major constituent of the extracellular matrix and is associated with the constrictive remodeling process of angioplastied arteries. Lafont et al., Circulation, Vol. 100, pp. 1109-1115 (1999). In addition, Meyers et al., J. Steroid Biochem., Vol. 14, pp. 1157-1168 (1981), has reported that mineralocorticoid receptors are present on smooth muscle cells.

The role of the mineralocorticoid aldosterone in the pathogenesis of neointimal thickening after PTCA has been the subject of several animal studies. In rabbits, neointimal thickening after balloon denudation was reportedly stimulated by aldosterone and reduced by the aldosterone receptor inhibitor spironolactone at a daily dosage of 50 mg/kg body weight. Van Belle et al., Cardiovascular Res., Vol. 29, pp. 27-32 (1995). *In vivo* administration of spironolactone also was reported to inhibit basic fibroblast growth factor (bFGF)-induced angiogenesis in rabbits in a chorioallantoic membrane ("CAM") assay. Klauber et al., Circulation, Vol. 94, pp. 2566-2571 (1996). *In vitro,* spironolactone was further reported (1) to inhibit bFGF and vascular endothelial growth factor-stimulated capillary endothelial cell proliferation; (2) to inhibit bFGF-stimulated capillary endothelial cell chemotaxis; and (3) to cause avascular zones when placed on the chick CAM. Klauber et al., Circulation, Vol. 94, pp. 2566-2571 (1996).

Rodriguez et al., Rev. Med. Chil., Vol. 125, pp. 643-652 (1997), has reported that spironolactone treatment for six months following acute myocardial infarction positively affected ventricular function as demonstrated by an improved ejection fraction.

The involvement of angiotensin converting enzyme ("ACE") in restenosis also has been reported in the literature. ACE is present in vascular intima and subintima of normal and injured vascular walls. Angiotensin II is believed to stimulate the release of PDGF, MDGF and FDGF, cytokines that are known stimulators of smooth muscle cell growth and proliferation *in vitro.* Direct blockade of the conversion of angiotensin I to angiotensin II by treatment with the ACE inhibitor captopril has been reported to effectively inhibit smooth muscle cell proliferation and restenosis post-angioplasty in a porcine organ culture model of coronary artery in vitro. Wilson et al., "Angiotensin II receptor antagonists prevent neointimal proliferation in a porcine coronary artery organ culture model", Cardiovasc. Res., Vol. 42(3), pp. 761-772 (June 1999).

ACE inhibition, exclusive of flow, does not attenuate proliferative restenosis. Furthermore, ACE is a secretagogue for angiotensin II. Nonetheless, inhibitors of smooth muscle cell proliferation have been mostly unsuccessful in preventing restenosis. Currier et al., J. Am. Coll. Cardiol., Vol. 25, pp. 515-520 (1995). Likewise, blockade of ACE alone by cilazapril in man in the MERCATOR trial also had no significant impact. Yamabe et al., Coron. Artery Dis., Vol. 6(7), pp. 573-579 (July 1995).

The mechanisms of, and potential treatments for, restenosis have been studied in experimental models of balloon denudation in rats and rabbits. ACE inhibitors were reported to reduce restenosis after balloon denudation in rats. Powell et al., Science, Vol. 245. pp. 186-188 (1989). ACE inhibitors also were reported to reduce restenosis after balloon denudation in rabbits. J. Am. Coll. Cardiol., Vol. 23, pp. 395A. Neointimal thickening and smooth muscle proliferation following balloon denudation is reportedly increased by angiotensin II administration. Daemen et al., Circ. Res., Vol. 68, pp. 450-456 (1991).

Treatment regimens employing administration of ACE inhibitors in patients shortly after myocardial infarction have been reported to reduce mortality. Cody, Arch. Intern. Med., Vol. 154, pp. 2029-2036 (1994). Hyperaldosteronism also has been associated with myocardial infarction. Denis et al., Arch. Mal. Coeur Vaiss, 77 Spec No:35-40 (1984). Hypertension can lead to vascular damage disease associated with glomerular and vascular lesions characteristic of thrombotic microangiopathy. ACE inhibitors or spironolactone markedly reduced proteinuria and malignant nephrosclerotic lesions in these animals. Rocha et al., Hypertension, Vol. 33 (1 Pt 2), pp. 232-7 (Jan. 1999).

Vascular damage resulting from hypertension can also lead to end-organ damage including stroke, cardiac hypertrophy, renal dysfunction, glomerulosclerosis, and/or vascular hypertrophy. In the stroke-prone spontaneously hypertensive rats model, angiotensin I receptor antagonists (eg, candesartan, cilexetil, losartan) reduced the incidence of stroke and renal injury even at doses which had no effect on blood pressure and effectively prevent the associated increases in TGF-beta1 and extracellular matrix components (fibronectin, collagen type I, III and IV and laminin). Nishikawa, Hum. Hypertens., Vol. 12(5), pp. 301-309 (May 1998).

There is also evidence in animal models to suggest that ACE inhibition is effective in reduction of arterial damage due to experimental hyperlipidemia. Lee et al., Vasc. Med., Vol. 1(2), pp. 109-113 (1996).

Radiation therapy can cause vascular damage and collagen deposition. In one report of a study in rats receiving total body irradiation followed by syngeneic bone marrow transplant, there was a marked reduction of glomerular, tubular, vascular, and interstitial damage in captopril-treated animals, with only mild focal tubular interstitial injury and fibrosis seen. There was also a reduction in the arteriolar wall thickening, luminal occlusion, and collagen deposition in irradiated, captopril-treated animals. Cohen et al., Lab. Invest., Vol. 75(3), pp. 349-360 (Sept. 1996).

### SUMMARY OF THE INVENTION

The invention provides the use of eplerenone for the preparation of a pharmaceutical composition for treating, inhibiting or preventing restenosis resulting from angioplasty of a blood vessel in a human subject wherein the composition contains eplerenone in an amount to administer a daily dose range of 0.5 mg to 500 mg.

The invention also provides a stent comprising eplerenone.

Furthermore, the invention provides the use of eplerenone for the preparation of a stent for treating, inhibiting or preventing restenosis of a blood vessel in a human subject.

Other aspects of the invention will be apparent and in part pointed out hereinafter.

### BRIEF DESCRIPTION OF THE DRAWINGS

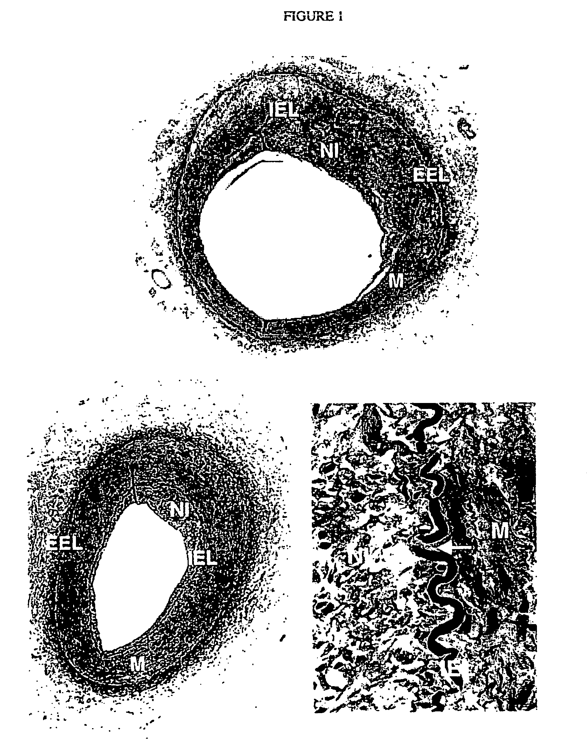

Figure 1 shows Masson's trichrome with Orchein stained cross-sections of coronary (top panel) and circumflex iliac (lower panels) arteries 28 days after angioplasty. The right lower panel shows the small fractures (see arrows) that occur in the internal elastic lamina of the angioplastied circumflex iliac arteries (seen only at high magnification). The internal elastic lamina ("IEL"), external elastic lamina ("EEL") and other laminae are stained black. L indicates the lumen, M the media and I the neointimal lesion.

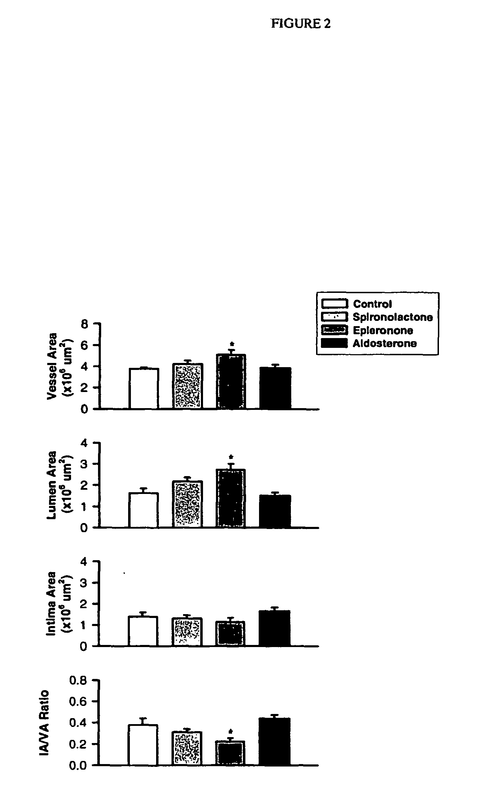

Figure 2 graphically shows the coronary artery (1) vessel area as defined by the EEL, (2) lumen cross-sectional area, (3) intima area, and (4) intima area/vessel area (IA/VA) ratio 28 days after angioplasty in animals receiving spironolactone, epleronone, aldosterone or no treatment (placebo). Results are means ± SEM. * indicates P < 0.05 from no treatment (control) group.

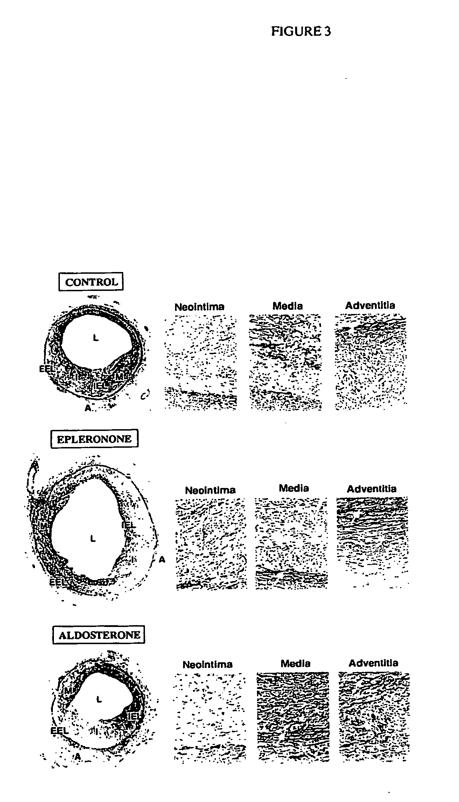

Figure 3 shows photomicrographs of coronary arteries 28 days after angioplasty that illustrate the density of collagen (green) and elastin (dark blue/black) in different regions of the coronary arteries of animals receiving no treatment (placebo), epleronone or aldosterone. Arrows identify the EEL of each vessel section and A represents the adventitia, IEL the internal elastic lamina, M the media and L the lumen.

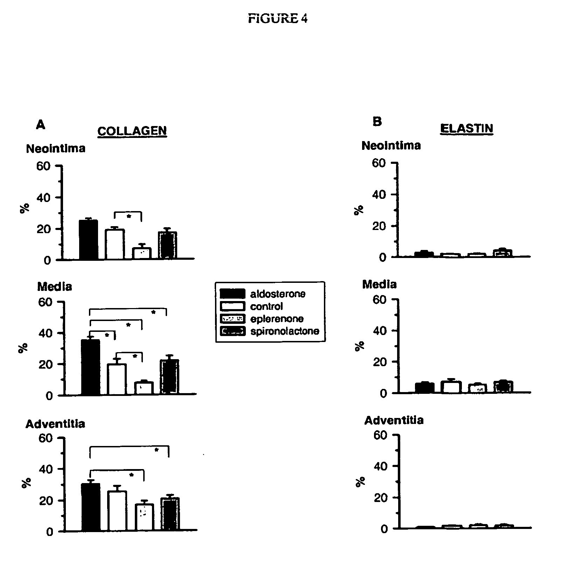

Figure 4A shows collagen content in the (1) neointima, (2) media, and (3) adventitia 28 days after angioplasty of coronary arteries of animals receiving aldosterone, no treatment (placebo), epleronone or spironolactone. Results are means ± SEM. * indicates P < 0.05 from no treatment (control) group.
Figure 4B shows elastin content in the (1) neointima, (2) media, and (3) adventitia 28 days after angioplasty for different regions of coronary arteries from the same groups of animals as Figure 4A. Results are means ± SEM. * indicates P < 0.05 from no treatment (control) group.

### DETAILED DESCRIPTION OF THE PREFERRED EMBODIMENTS

The present invention relates to methods (not claimed as such) for treating, inhibiting or preventing pathogenic change resulting from vascular injury in a subject, namely restenosis resulting from angioplasty. The methods comprise administering an aldosterone antagonist, namely eplerenone, to a mammalian subject susceptible to or suffering from pathogenic change resulting from vascular injury, wherein the aldosterone antagonist is administered in an amount that is therapeutically effective in suppressing the pathogenic change.

The phrase "vascular injury" as used in this application includes to damage to the vasculature of a subject resulting from and secondary to, for example, trauma, or surgery as distinguished from damage to the vasculature resulting solely from arteriosclerotic or atherosclerotic vascular disease. Vascular injury also can result, for example, from an infarct. When there is a disruption of blood flow from, for example, a thrombus, ischemia from the disruption of blood flow can result in necrosis. A series of ventricular remodeling and cardiovascular events can ensue that involves mechanisms underlying restenosis, namely smooth muscle cell proliferation and cellular matrix production.

The phrase "pathogenic change resulting from vascular injury" includes a change in the vasculature that is not ordinarily associated with, or that exceeds, that change associated with the normal healing process that is necessary for restoration and repair of the vasculature. Pathogenic changes generally are constrictive in nature and can result, for example, in a decrease in the area encompassed by the external elastic lamina of an artery. Examples of pathogenic changes can include lumenal narrowing, restrictive neointima formation, vascular collagen accumulation, migration and proliferation of smooth muscle cells, and extracellular matrix production.

The phrase "trauma" includes a physical injury or wound caused by external force or violence including, injury resulting from a motor vehicle accident, a suicide attempt, a fall, burns or amputation.

The phrase "surgery" is used in accordance with its ordinary meaning and should be interpreted broadly. It includes surgery such as angioplasty, arterial resection, tissue reconstruction, tissue graft including venous, arterial and prosthetic materials, treatment of fractures and other damage to bones, repair of an arteriovenuous fistula, and digital replantation.

The phrase "angioplasty" includes the alteration of the structure of a vessel, either by dilating the vessel using a ballon inside the lumen or by other surgical procedure. The term "angioplasty" includes percutaneous transluminal coronary angioplasty.

The term "subject" as used herein includes a mammal, preferably a human, who has been the object of treatment, observation or experiment.

The term "treatment" includes any process, action, application, therapy, or procedure, wherein a mammal, particularly a human, is subjected to medical aid with the object of improving the mammal's condition, directly or indirectly.

The term "prevention" includes either preventing the onset of a clinically evident pathologic change resulting from vascular injury altogether or preventing the onset of a preclinically evident stage of a pathologic change resulting from vascular injury in a subject. This term encompasses the prophylactic treatment of a subject at risk of developing a pathologic change resulting from vascular injury, including restenosis.

The term "inhibiting" includes slowing or stopping the progression of a clinically evident pathologic change resulting from vascular injury altogether or slowing or stopping the progression of the onset of a preclinically evident stage of a pathologic change resulting from vascular injury in a subject.

The phrase "therapeutically-effective" qualifies the amount of the aldosterone antagonist that will achieve the goal of improvement in condition or disorder while avoiding adverse side effects typically associated with alternative therapies.

The term "pharmaceutically acceptable" is used adjectivally herein to mean that the modified noun is appropriate for use in a pharmaceutical product. Pharmaceutically acceptable cations include metallic ions and organic ions. More preferred metallic ions include appropriate alkali metal salts, alkaline earth metal salts and other physiologically acceptable metal ions. Exemplary ions include aluminum, calcium, lithium, magnesium, potassium, sodium and zinc in their usual valences. Preferred organic ions include protonated tertiary amines and quaternary ammonium cations, including in part, trimethylamine, diethylamine, N,N'-dibenzylethylenediamine, chloroprocaine, choline, diethanolamine, ethylenediamine, meglumine (N-methylglucamine) and procaine. Exemplary pharmaceutically acceptable acids include hydrochloric acid, hydrobromic acid, phosphoric acid, sulfuric acid, methanesulfonic acid, acetic acid, formic acid, tartaric acid, maleic acid, malic acid, citric acid, isocitric acid, succinic acid, lactic acid, gluconic acid, glucuronic acid, pyruvic acid, oxalacetic acid, fumaric acid, propionic acid, aspartic acid, glutamic acid and benzoic acid.

The phrase "restenosis" includes the reoccurence of a stenosis condition in the vasculature of a subject, particularly the reoccurence of a stenosis condition treated by angioplasty.

The phrase "extracellular matrix" includes the extracellular components elaborated by smooth muscle cells and fibroblast-like cells of mesenchymal origin primarily comprising fibronectin, collagen, elastin and laminin.

The phrase "constrictive remodeling" includes a lumenal narrowing of an artery resulting in a pathophysiologic response to controlled arterial injury that involves smooth muscle cell proliferation and migration into the subintima and elaboration of matrix.

The phrase "restrictive neointima formation" includes a lumenal narrowing of blood vessel resulting in a pathophysiologic response to vascular injury, irrespective of the underlying mechanism.

The vascular injury is an injury to a vessel such as an artery, preferably a coronary artery or a pulmonary artery. More specifically, the injury is to a coronary artery and substantially caused by angioplasty and substantially resulting in the occurrence of restenosis.

In one embodiment wherein the vascular injury is injury to an artery, the eplerenone is administered in an amount effective to maintain, for a period of at least about one month, more preferably at least about six months, after the occurrence of the injury, the ratio of intima area to vessel area of the injured artery at the site of maximal injury below about 0.37, more preferably below about 0.35, still more preferably below about 0.33, still more preferably below about 0.31, and still more preferably below about 0.30. The injured artery in this embodiment preferably is a coronary artery injured substantially as a result of angioplasty.

The effectiveness of the present invention typically is more pronounced as the severity of the vascular injury increases. For arterial injuries that can be assessed using the gap angle of injury procedure set forth in Andersen et al., "Remodeling Rather Than Neointimal Formation Explains Luminal Narrowing After Deep Vessel Wall Injury: Insights From A Porcine Coronary (Re)stenosis Model", Circulation, Vol. 93, pp. 1716-1724 (1996), the arterial injury preferably is one having a gap angle of injury at the site of maximal injury to the artery that is at least about 10°, preferably at least about 20°, more preferably at least about 30°, still more preferably at least about 40°, and still more preferably at least about 50°.

The present invention is useful for human subjects as well as mammalian companion animals, exotic animals and farm animals. The subject preferably is a mammal such as a human, dog, cat or horse, and most preferably is a human.

Without being held to a particular theory or mechanism, it is hypothesized that aldosterone antagonists reduce extracellular matrix production in injured vessels and retard or prevent neointimal thickening after angioplasty.

### Aldosterone Antagonists

The phrase "aldosterone antagonist" embraces an agent or compound, or a combination of two or more of such agents or compounds, that counteract the effect of aldosterone. Such agents and compounds, such as mespirenone, may antagonize the action of aldosterone through pre-receptor mechanism. Other agents and compounds, such as eplerenone and spironolactone, fall generally within a class known as aldosterone receptor antagonists and bind to aldosterone receptors such as are typically found in renal tubules, and prevent natural ligand activation of post-receptor events.

The term "spirolactone-type" is intended to characterize a structure comprising a lactone moiety attached to a steroid nucleus, typically at the steroid "D" ring, through a spiro bond configuration. A subclass of spirolactone-type aldosterone antagonist compounds consists of epoxy-steroidal aldosterone antagonist compounds such as eplerenone. Another subclass of spirolactone-type antagonist compounds consists of non-epoxy-steroidal aldosterone antagonist compounds such as spironolactone.

The epoxy-steroidal aldosterone antagonist compounds used in the method of the present invention generally have a steroidal nucleus substituted with an epoxy-type moiety. The term "epoxy-type" moiety is intended to embrace any moiety characterized in having an oxygen atom as a bridge between two carbon atoms, examples of which include the following moieties:

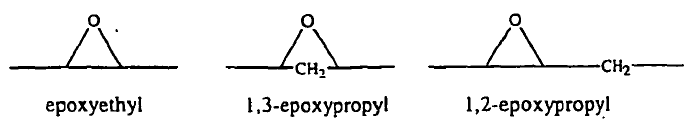

The term "steroidal", as used in the phrase "epoxy-steroidal", denotes a nucleus provided by a cyclopemeno-phenanthrene moiety, having the conventional "A", "B", "C" and "D" rings. The epoxy-type moiety may be attached to the cyclopentenophenanthrene nucleus at any attachable or substitutable positions, that is, fused to one of the rings of the steroidal nucleus or the moiety may be substituted on a ring member of the ring system. The phrase "epoxy-steroidal" is intended to embrace a steroidal nucleus having one or a plurality of epoxy-type moieties attached thereto.

Epoxy-steroidal aldosterone antagonists include a family of compounds having an epoxy moiety fused to the "C" ring of the steroidal nucleus. 20-spiroxane compounds are characterized by the presence of a 9α,11α-substituted epoxy moiety. Compounds 1 through 11, below, are illustrative 9α,11α-epoxy-steroidal compounds.

These epoxy steroids may be prepared by procedures described in Grob et al., U.S. Patent No. 4,559,332. Additional processes for the preparation of 9,11-epoxy steroidal compounds and their salts are disclosed in Ng et al., WO97/21720 and Ng et al., WO98/25948.

**TABLE I: Aldosterone Receptor Antagonist**

| Compound # | Structure | Name |
|---|---|---|
| 1 | 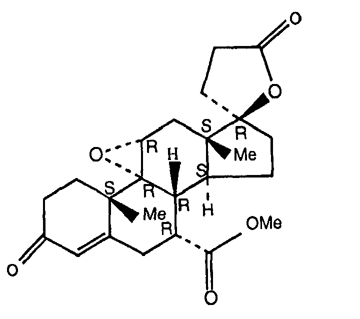 | Pregn-4-ene-7,21-dicarboxylic acid, 9,11-epoxy-17-hydroxy-3-oxo-,γ-lactone, methyl ester, (7α,11α,17β)- |
| 2 | 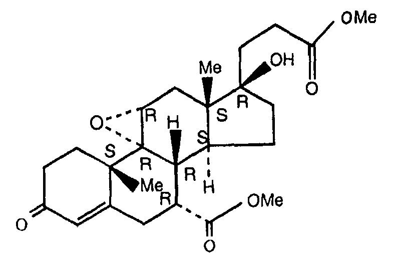 | Pregn-4-ene-7,21-dicarboxylic acid, 9,11-epoxy-17-hydroxy-3-oxo-,dimethyl ester, (7α,11α,17β)- |
| 3 | 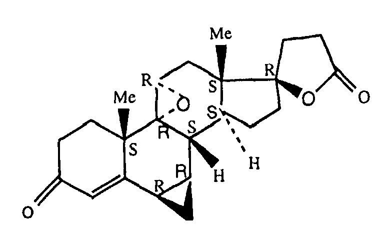 | 3'H-cyclopropa[6,7]pregna-4,6-diene-21-carboxylic acid, 9,11-epoxy-6,7-dihydro-17-hydroxy-3-oxo-, γ-lactone, (6β,7β,11α,17β)- |
| 4 | 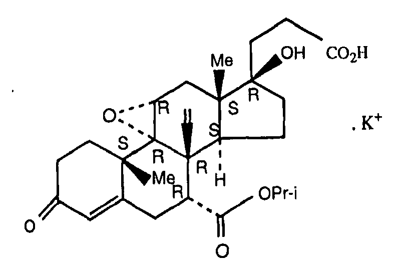 | Pregn-4-ene-7,21-dicarboxylic acid,9,11-epoxy-17-hydroxy-3-oxo-,7-(1-methylethyl) ester, monopotassium salt, (7α,11α,17β)- |
| 5 | 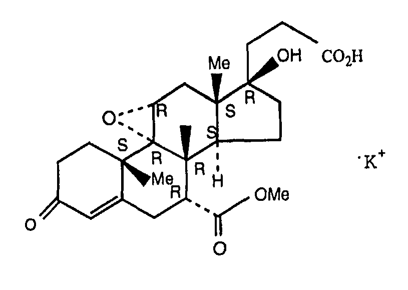 | Pregn-4-ene-7,21-dicarboxylic acid, 9,11-epoxy-17-hydroxy-3-oxo-,7-methylethyl) ester,monopotassium salt, (7α,11α,17β)- |
| 6 | 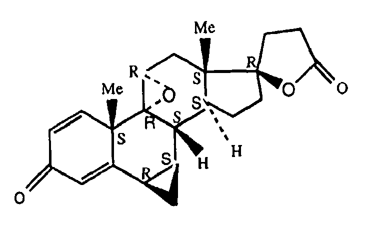 | 3'H-cyclopropa[6,7]pregna-1,4,6-triene-21-carboxylic acid,9,11-epoxy-6,7-dihydro-17-hydroxy-3-oxo-, γ-lactone (6β,7β,11α)- |
| 7 | 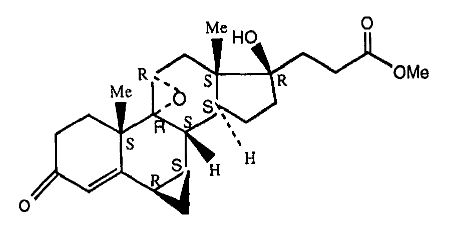 | 3'H-cyclopropa[6,7]pregna-4,6-diene-21-carboxylic acid, 9,11-epoxy-6,7-dihydro-17-hydroxy-3-oxo-, methyl ester, (6β,7β,11α,17β)- |
| 8 | 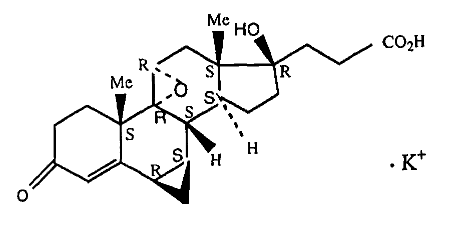 | 3'H-cyclopropa[6,7]pregna-4,6-diene-21-carboxylic acid, 9,11-epoxy-6,7-dihydro-17-hydroxy-3-oxo-, monopotassium salt, (6β,7β,11α,17β)- |
| 9 | 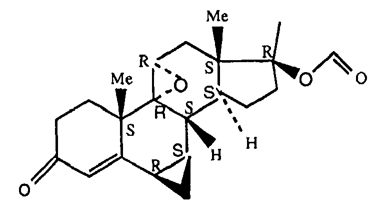 | 3'H-cyclopropa[6,7]pregna-1,4,6-triene-21-carboxylic acid, 9,11-epoxy-6,7-dihydro-17-hydroxy-3-oxo-,γ-lactone (6β,7β,11α,17β)- |
| 10 | 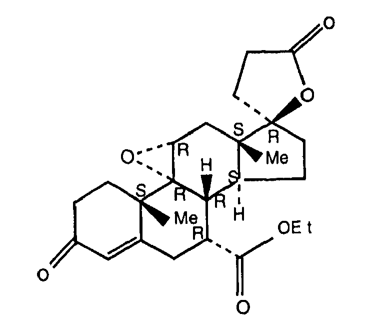 | Pregn-4-ene-7,21-dicarboxylic acid, 9,11-epoxy-17-hydroxy-3-oxo-,γ-lactone, ethyl ester, (7α,11α,17β)- |
| 11 | 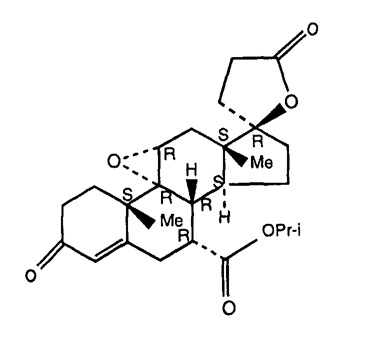 | Pregn-4-ene-7,21-dicarboxylic acid, 9,11-epoxy-17-hydroxy-3-oxo-,γ-lactone, 1-methylethyl ester (7α,11α,17β)- |

The compound eplerenone (also known as epoxymexrenone) is compound 1 as shown above. Eplerenone is an aldosterone receptor antagonist and has a higher specificity for aldosterone receptors than does, for example, spironolactone. Selection of eplerenone as the aldosterone antagonist in the present method is beneficial to reduce certain side-effects such as gynecomastia that occur with use of aldosterone antagonists having less specificity.

Non-epoxy-steroidal aldosterone antagonists include a family of spirolactone-type compounds defined by Formula I: 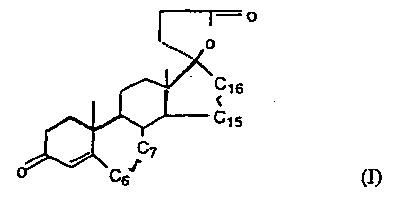 wherein 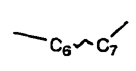 is 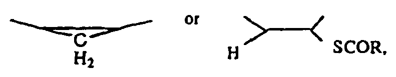 wherein R is lower alkyl of up to 5 carbon atoms, and
wherein 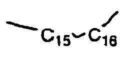 is

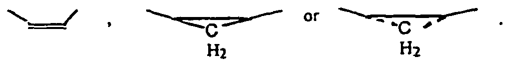

Lower alkyl residues include branched and unbranched groups, preferably methyl, ethyl and n-propyl.

Specific compounds of interest within Formula I are the following:
7α-acetylthio-3-oxo-4,15-androstadiene-[17(β-1')-spiro-5']perhydrofuran-2'-one:
3-oxo-7α-propionylthio-4,15-androstadiene-[17((β-1')-spiro-5']perhydrofuran-2'-one;
6β,7β-methylene-3-oxo4,15-androstadiene-[17((β-1')-spiro-5']perhydrofuran-2'-one;
15α,16α-methylene-3-oxo-4,7α-propionylthio-4-androstene[17(β-1')-spiro-5']perhydrofuran-2'-one;
6β,7β,15α,16α-dimethylene-3-oxo-4-androstene[17(β-1')-spiro-5']-perhydrofuran-2'-one;
7α-acetylthio-15β,16β-Methylene-3-oxo-4-androstene-[17(β-1')-spiro-5']perhydrofuran-2'-one;
15β,16β-methylene-3-oxo-7β-propionylthio-4-androstene-[17(β-1')-spiro-5']perhydrofuran-2'-one; and
6β,7β,15β,16β-dimethylene-3-oxo-4-androstene-[17(β-1')-spiro-5']perhydrofuran-2'-one.

Methods to make compounds of Formula I are described in U.S. Patent No. 4,129,564 to Wiechart et al. issued on 12 December 1978.

Another family of non-epoxy-steroidal compounds of interest is defined by Formula II: 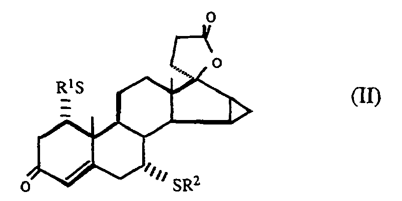 wherein R¹ is C₁₋₃-alkyl or C₁₋₃ acyl and R² is H or C₁₋₃-alkyl.

Specific compounds of interest within Formula II are the following:
1α-acetylthio-15β,16β-methylene-7α-methylthio-3-oxo-17α-pregn-4-ene-21,17-carbotactone; and
15β,16β-methylene-1α,7α-dimethylthio-3-oxo-17α-pregn-4-ene-21,17-carbolactone.

Methods to make the compounds of Formula II are described in U.S. Patent No. 4,789,668 to Nickisch et al. which issued 6 December 1988.

Yet another family of non-epoxy-steroidal compounds of interest is defined by a structure of Formula III: 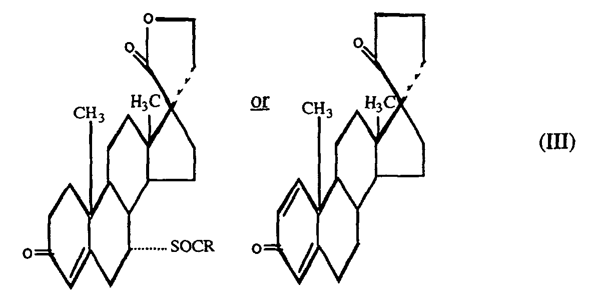 wherein R is lower alkyl, with preferred lower alkyl groups being methyl, ethyl, propyl and butyl. Specific compounds of interest include:
3β,21-dihydroxy-17α-pregna-5,15-diene-17-carboxylic acid γ-lactone;
3β,21-dihydroxy-17α-pregna-5,15-diene-17-carboxylic acid γ-lactone 3-acetate;
3β,21-dihydroxy-17α-pregn-5-ene-17-carboxylic acid γ-lactone;
3β,21-dihydroxy-17α-pregn-5-ene-17-carboxylic acid γ-lactone 3-acetate;
21-hydroxy-3-oxo-17α-pregn-4-ene-17-carboxylic acid γ-lactone;
21-hydroxy-3-oxo-17α-pregna-4,6-diene-17-carboxylic acid γ-lactone;
21-hydroxy-3-oxo-17α-pregna-1,4-diene-17-carboxylic acid γ-lactone;
7α-acylthio-21-hydroxy-3-oxo-17α-pregn-4-ene-17-carboxylic acid γ-lactone; and
7α-acetylthio-21-hydroxy-3-oxo-17α-pregn-4-ene-17-carboxylic acid γ-lactone.

Methods to make the compounds of Formula III are described in U.S. Patent No. 3,257,390 to Patchett which issued 21 June 1966.

Still another family of non-epoxy-steroidal compounds of interest is represented by Formula IV: 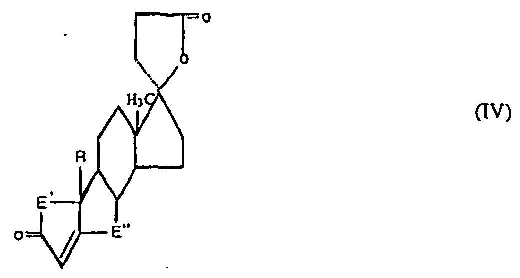 wherein E' is selected from the group consisting of ethylene, vinylene and (lower alkanoyl)thioethylene radicals, E" is selected from the group consisting of ethylene, vinylene, (lower alkanoyl)thioethylene and (lower alkanoyl)thiopropylene radicals; R is a methyl radical except when E' and E" are ethylene and (lower alkanoyl) thioethylene radicals, respectively, in which case R is selected from the group consisting of hydrogen and methyl radicals; and the selection of E' and E" is such that at least one (lower alkanoyl)thio radical is present.

A family of non-epoxy-steroidal compounds within Formula IV is represented by Formula V:

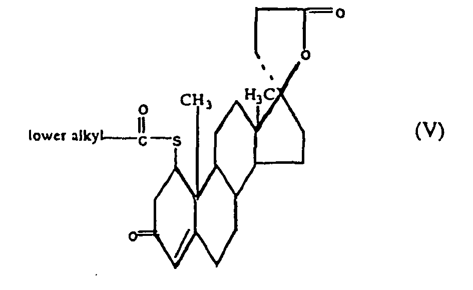

A compound of Formula V is 1-acetylthio-17α-(2-carboxyethyl)-17β-hydroxy-androst-4-en-3-one lactone.

Another family of non-epoxy-steroidal compounds within Formula IV is represented by Formula VI:

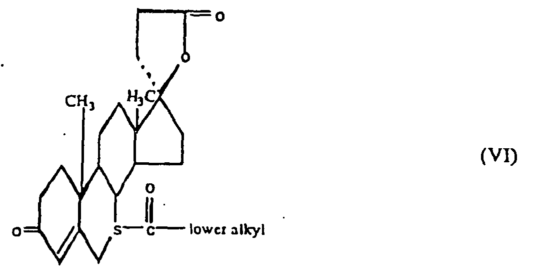

Compounds within Formula VI include the following:
7α-acetylthio-17α-(2-carboxyethyl)-17β-hydroxy-androst-4-en-3-one lactone;
7β-acetylthio-17α-(2-carboxyethyl)-17β-hydroxy-androst-4-en-3-one lactone;
1α,7α-diacetylthio-17α-(2-carboxyethyl)-17β-hydroxy-androsta-4,6-dien-3-one lactone;
7α-acetylthio-17α-(2-carboxyethyl)-17β-hydroxy-androsta-1,4-dien-3-one lactone;
7α-acetylthio-17α-(2-carboxyethyl)-17β-hydroxy-19-norandrost-4-en-3-one lactone; and
7α-acetylthio-17α-(2-carboxyethyl)-17β-hydroxy-6α-methylandrost-4-en-3-one lactone;

In Formulae IV-VI, the term "alkyl" is intended to embrace linear and branched alkyl radicals containing one to about eight carbons. The term "(lower alkanoyl)thio" embraces radicals of the formula lower alkyl

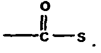

Of particular interest is the compound spironolactone having the following structure and formal name: 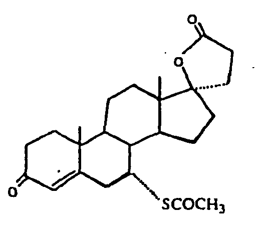 "spironolactone": 17-hydroxy-7α-mercapto-3-oxo-17α-pregn-4-ene-21-carboxylic acid γ-lactone acetate.

Methods to make compounds of Formulae RV-VI are described in.U.S. Patent No. 3,013,012 to Cella et al. which issued 12 December 1961. Spironolactone is sold by G.D. Searle & Co., Skokie, Illinois, under the trademark "ALDACTONE", in tablet dosage form at doses of 25 mg. 50 mg and 100 mg per tablet.

### Dosages and Treatment Regimen

The amount of aldosterone antagonist (i.e., eplerenone) that is administered and the dosage regimen for the methods of this invention depend on a variety of factors, including the age, weight, sex and medical condition of the subject, the severity of the disease, the route and frequency of administration, and thus may vary widely. A daily dose administered to a subject of about 0.001 to 30 mg/kg body weight, preferably between about 0.005 and about 20 mg/kg body weight, more preferably between about 0.01 and about 15 mg/kg body weight, still more preferably between about 0.05 and about 10 mg/kg body weight, and most preferably between about 0.01 to 5 mg/kg body weight, may be appropriate. The amount of aldosterone antagonist that is administered to a human subject ranges from 0.5 to 500 mg, and more preferably from 1 to 100 mg. A daily dose of aldosterone antagonist that produces no substantial, diuretic effect in a subject is specifically embraced by the present invention. The daily dose can be administered in one to four doses per day.

It can be beneficial, particularly where the vascular injury results substantially from surgery, to begin the administration of the aldosterone antagonist, for example, prior to surgery and to continue administration of the aldosterone antagonist after surgery for a sufficient period of time. Such pretreatment of the subject with the aldosterone antagonist is desirable particularly where the aldosterone antagonist is administered to treat, inhibit or prevent restenosis of an artery substantially resulting from angioplasty of that artery.

### Dosing based on Natriuretic Peptides and PIIINP

The natriuretic peptides are a group of structurally similar but genetically distinct peptides that have diverse actions in cardiovascular, renal, and endocrine homeostasis. Atrial natriuretic peptide ("ANP") and brain natriuretic peptide ("BNP") are of myocardial cell origin and C-type natriuretic peptide ("CNP") is of endothelial origin. ANP and BNP bind to the natriuretic peptide-A receptor ("NPR-A"), which, via 3',5'-cyclic guanosine monophosphate ("cGMP"), mediates natriuresis, vasodilation, renin inhibition, antimitogenesis, and lusitropic properties. Elevated natriuretic peptide levels in the blood, particularly blood BNP levels, generally are observed in subjects after vascular injury such as acute myocardial infarction and remain elevated for an extended period of time after the infarction. Uusimaa et al., "Plasma vasoactive peptides after acute myocardial infarction in relation to left ventricular dysfunction", Int. J. Cardiol., Vol. 69(1), pp. 5-14 (April 30, 1999).

Extracellular matrix turnover is one of the determinants of vascular constrictive remodeling and may be monitored by measuring the blood level of procollagen type III aminoterminal propeptide ("PIIINP"). For example, in congestive heart failure the extracellular matrix turnover is a major determinant of cardiac remodeling, diastolic function and pumping capacity.

Accordingly, dosing of the aldosterone antagonist may be determined and adjusted based on measurement of blood concentrations of one or more of PIIINP, ANF, ANP and/or BNP. A decrease in blood PIIINP level relative to baseline PIIINP level prior to administration of the aldosterone antagonist, for example, indicates a decrease in extracellular matrix turnover and therefore provides a correlation with inhibition of vascular constrictive remodeling. Similarly, blood levels of ANF, ANP and/or BNP may be compared against the corresponding baseline levels prior to administration of the aldosterone antagonist to determine efficacy of the present method.

### Pharmaceutical Compositions:

The aldosterone antagonist (i.e., eplerenone) may be administered in the form of a pharmaceutical composition. For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. The active ingredient also may be administered by injection as a composition wherein, for example, saline, dextrose or water may be used as a suitable carrier.

Other formulations may be in the form of a topical ointment or cream, or a suppository, containing the active ingredients in a total amount of, for example, 0.075 to 30% w/w, preferably 0.2 to 20% w/w and most preferably 0.4 to 15% w/w. When formulated in an ointment, the active ingredients may be employed with either paraffinic or a water-miscible ointment base. Alternatively, the active ingredients may be formulated in a cream with an oil-in-water cream base. If desired, the aqueous phase of the cream base may include, for example at least 30% w/w of a polyhydric alcohol such as propylene glycol, butane-1,3-diol, mannitol, sorbitol, glycerol, polyethylene glycol and mixtures thereof. The topical formulation may desirably include a compound which enhances absorption or penetration of the active ingredient through the skin or other affected areas. Examples of such dermal penetration enhancers include dimethylsulfoxide and related analogs.

The aldosterone antagonist also can be administered by a transdermal device. Preferably, topical administration will be accomplished using a patch either of the reservoir and porous membrane type or of a solid matrix variety. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

The oily phase of the emulsions of this invention may be constituted from known ingredients in a known manner. While the phase may comprise merely an emulsifier, it may comprise a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make-up the so-called emulsifying wax, and the wax together with the oil and fat make up the so-called emulsifying ointment base which forms the oily dispersed phase of the cream formulations. Emulsifiers and emulsion stabilizers suitable for use in the formulation of the present invention include Tween 60, Span 80, cetostearyl alcohol, myristyl alcohol, glyceryl monostearate, and sodium lauryl sulfate, among others.

The choice of suitable oils or fats for the formulation is based on achieving the desired cosmetic properties, since the solubility of the active compound in most oils likely to be used in pharmaceutical emulsion formulations is very low. Thus, the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters may be used. These may be used alone or in combination depending on the properties required. Alternatively, high melting point lipids such as white soft paraffin and/or liquid paraffin or other mineral oils can be used.

For therapeutic purposes, the aldosterone antagonist ordinarily is combined with one or more adjuvants appropriate to the indicated route of administration. If administered per os, the compound may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The solid state forms of eplerenone may be dissolved in water, polyethylene glycol, propylene glycol, ethanol, com oil, cottonseed oil, peanut oil, sesame oil, benzyl alcohol, sodium chloride, and/or various buffers. Other adjuvants and modes of administration are well and widely known in the pharmaceutical art.

### Combination Therapies

Other active ingredients or therapies may be administered in combination with the administration of the aldosterone antagonist (i.e., eplerenone). For example, conventional treatment of restenosis resulting from angioplasty includes therapies such as exposing the artery at the site of injury to a source of radiation to inhibit restrictive neointima growth and inserting an endolumenal stent at the site of angioplasty. Administration of the aldosterone antagonist can be effected in combination with one or more of these conventional treatments where desirable.

For example, the aldosterone antagonist can be administered in combination with exposure of the artery at the site of injury to a source of radiation to inhibit restrictive neointima growth. Although radiation monotherapy has been used to prevent restenosis after angioplasty, Powers et al., Int. J. Radial. Oncol. Biol, Vol. 45(3), pp. 753-759 (Oct. 1. 1999), report findings in a study involving a canine model that indicate that adventitial fibrosis increases with increasing dose of radiation and can contribute to adverse late vascular remodeling. The proposed combination therapy would permit the use of dosages of radiation below conventional monotherapeutic dosages of radiation and would result in fewer side-effects or adverse effects relative to such radiation monotherapy.

In another embodiment, the stent itself comprises the aldosterone antagonist and is used as a carrier to effect local delivery of the aldosterone antagonist to the injured vessel. The aldosterone antagonist is coated on, adsorbed on, affixed to or present on the surface of the stent or is otherwise present in or on the matrix of the stent, either alone or in combination with other active drugs and pharmaceutically acceptable carriers, adjuvants and binding agents .The stent preferably comprises the aldosterone antagonist in the form of an extended release composition that provides for release of the antagonist over an extended period of time.

Additional illustrative combination therapies include the administration of other active drugs used in the treatment of cardiovascular-related conditions and disorders in combination with the aldosterone antagonists employed in the present methods. The active drugs administered with the aldosterone antagonist can include, for example, the drugs selected from the group consisting of renin inhibitors, angiotensin **I** antagonists, angiotensin II antagonists, angiotensin converting enzyme inhibitors, diuretics having no substantial aldosterone antagonist effect, and retinoic acid. The phrase "combination therapy" (or "co-therapy"), when used with respect to drug combinations, is intended to embrace the administration of each agent in a sequential manner in a regimen that will provide beneficial effects of the drug combination, and is intended as well to embrace coadministration of these agents in a substantially simultaneous manner, such as in a single capsule or injection having a fixed ratio of these active agents or in multiple, separate capsules or injections for each agent.

The phrase "angiotensin II antagonists" includes, for examples, those angiotensin II antagonists described in WO96/40257.

The phrase "angiotensin converting enzyme inhibitor" ("ACE inhibitor") is intended to embrace an agent or compound, or a combination of two or more agents or compounds, having the ability to block, partially or completely, the enzymatic conversion of the decapeptide form of angiotensin ("Angiotensin I") to the vasoconstrictive octapeptide form of angiotensin ("Angiotensin II"). Blocking the formation of Angiotensin II can affect the regulation of fluid and electrolyte balance, blood pressure and blood volume by removing the primary actions of Angiotensin II. Included in these primary actions of Angiotensin II are stimulation of the synthesis and secretion of aldosterone receptor by the adrenal cortex and raising blood pressure by direct constriction of the smooth muscle of the arterioles.

Examples of ACE inhibitors that can be used in the combination therapy include the following compounds: AB-103, ancovenin, benazeprilat, BRL-36378, BW-A575C, CGS-13928C, CL-242817, CV-5975, Equaten, EU-4865, EU-4867, EU-5476, foroxymithine, FPL 66564, FR-900456, Hoe-065, I5B2, indolapril, ketomethylureas, KRI-1177, KRI-1230, L-681176, libenzapril, MCD, MDL-27088, MDL-27467A, moveltipril, MS-41, nicotianamine, pentopril, phenacein, pivopril, rentiapril, RG-5975, RG-6134, RG-6207, RGH-0399, ROO-911, RS-10085-197, RS-2039, RS 5139, RS 86127, RU-44403, S-8308, SA-291, spiraprilat, SQ-26900, SQ-28084, SQ-28370, SQ-28940, SQ-31440, Synecor, utibapril, WF-10129, Wy-44221, Wy-44655, Y-23785, Yissum P-0154, zabicipril, Asahi Brewery AB-47, alatriopril, BMS 182657, Asahi Chemical C-111, Asahi Chemical C-112, Dainippon DU-1777, mixanpril, Prentyl, zofenoprilat, 1-(-(1-carboxy-6-(4-piperidinyl)hexyl)amino)-1-oxopropyl octahydro-1H-indole-2-carboxylic acid, Bioproject BP1.137, Chiesi CHF 1514, Fisons FPL-66564, idrapril, Marion Merrell Dow MDL-100240, perindoprilat and Servier S-5590, alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

A group of ACE inhibitors of particular interest consists of alacepril, benazepril, captopril, cilazapril, delapril, enalapril, enalaprilat, fosinopril, fosinoprilat, imidapril, lisinopril, perindopril, quinapril, ramipril, saralasin acetate, temocapril, trandolapril, ceranapril, moexipril, quinaprilat and spirapril.

Many of these ACE inhibitors are commercially available. For example, a highly preferred ACE inhibitor, captopril, is sold by E.R. Squibb & Sons, Inc., Princeton, NJ., now part of Bristol-Myers-Squibb, under the trademark "CAPOTEN", in tablet dosage form at doses of 12.5 mg, 50 mg and 100 mg per tablet. Enalapril or Enalapril Maleate, and Lisinopril are two more highly preferred ACE inhibitors sold by Merck & Co, West Point, Pa. Enalapril is sold under the trademark "VASOTEC" in tablet dosage form at doses of 2.5 mg, 5 mg, 10 mg and 20 mg per tablet. Lisinopril is sold under the trademark "PRINIVIL" in tablet dosage form at doses of 5 mg, 10 mg, 20 mg and 40 mg per tablet.

The diuretic may be selected from several known classes, such as thiazides and related sulfonamides, potassium-sparing diuretics, loop diuretics and organic mercurial diuretics. Examples of thiazides are bendroflumethiazide, benzthiazide, chlorothiazide, cyclothiazide, hydrochlorothiazide, hydroflumethiazide, methylclothiazide, polythiazide and trichlormethiazide. Examples of sulfonamides related to thiazides are chlorthalidone, quinethazone and metolazone. Examples of potassium-sparing diuretics are triameterene and amiloride. Examples of loop diuretics, i.e., diuretics acting in the ascending limb of the loop of Henle of the kidney, are furosemide and ethynacrylic acid. Examples of organic mercurial diuretics are mercaptomerin sodium, merethoxylline, procaine and mersalyl with theophylline.

The following example contains a detailed description of the method of the present invention. This detailed description falls within the scope of, and serves to exemplify, the invention.

### EXAMPLE 1

### Animals, Surgical Procedures and Drug Treatments

Mature male Boston mini-pigs (26-40 weeks old, 40-60 kg), were obtained from Monash University, Clayton, Australia. Injury was inflicted on one or two coronary artery branches and one or two iliac artery branches in each pig. The iliac arteries injured were the right and/or left recurrent circumflex branch of the external iliac artery, which is similar in size to the coronary arteries (about 2.5 to 3.0 mm diameter). Two of the three main coronary branches (the right coronary artery, the left anterior descending and left circumflex coronary arteries), were dilated in each pig, except when difficulties were encountered due to poor catheter engagement or arrhythmias.

All animals were administered 300 mg aspirin per day orally, starting seven days prior to the initial procedures and continuing for the duration of the study. Verapamil (120 mg per os, Knoll, Lane Cove, Australia) was administered in the 12 hour period before surgery. Immediately prior to surgery, the pigs were premedicated with acepromazine (0.1 mg/kg i.m., Delta, Hornsby, Australia) and atropine sulphate (1.2 mg i.m., Delta West, Bentley, Australia). Anesthesia was induced with propofol (150-200 mg i.v., ICl, Melbourne, Australia), and then maintained with inhaled isofluorane (Abbott, Kurnell, Australia).

Subcutaneous transluminal angioplasty was performed on the pigs using an 8F JL4 guiding catheter through a sheath inserted into the right common carotid artery, after intravenous heparin (15,000 units, Fissons, Thornleigh, Australia). Angiography was then performed after intra-arterial administration of glyceryl trinitrate (200 µg, Fissons, Thornleigh, Australia) using ioxaglate (Hexabrix, Mallinckrodt, Notting Hill, Australia) as the contrast medium. Recording was carried out in the left anterior oblique view (25°) for the coronary arteries and in the straight view for the iliac vessels.

The arteries were then injured using standard human angioplasty catheters (semicompliant, 20 mm length) that were oversized according to the manufacturer-specified balloon size with a balloon:artery ratio of 1.3-1.5:1. The balloon catheter was inflated to 10 atmospheres for 30 seconds with three separate inflations separated by one-minute reperfusion periods in both the coronary and the iliac vessels. To easily identify the injured segments when harvesting the vessels, the most proximal segment of each artery was injured. Angiography under anesthesia was repeated, immediately prior to euthanasia, on the coronary and iliac arteries 28 days later to confirm that the angioplastied vessels had remained patent.

Angioplasty was performed on four groups of pigs, each group containing six pigs. The first group was a control group that was untreated (control). The second group received 100 mg/day of epleronone orally in the morning. The third group received 200 mg/day of spironolactone in two divided oral doses. The fourth group received 400 µg/day aldosterone by continuous subcutaneous infusion. Treatment with epleronone and spironolactone commenced seven days prior to angioplasty and then continued for an additional 28 days after angioplasty. Aldosterone infusion was accomplished by placing two osmotic mini-pumps subcutaneously at the time of angioplasty. Aldosterone infusion continued for 28 days.

### Vessel Isolation and Processing for Histology

Animals were anesthetized, heparinized and then euthanized 28 days following angioplasty with ketamine and pentobarbitone. After the aorta, heart and the iliac vessels were exposed, the coronary and circumflex iliac artery were then perfused for five minutes with 4% formalin in phosphate buffered saline ("PBS"; pH 7.4) at 100 to 150 mm Hg. For the perfusion of the coronary vessels, the aorta was cross-clamped and the right atrial appendages were incised upon commencing the infusion of formalin through a cannula inserted into the aorta.

For the perfusion of the circumflex artery, the distal aorta, internal iliac arteries and the external iliac arteries distal to the recurrent circumflex branch were ligated to isolate the recurrent circumflex iliac artery. A cannula was then introduced into the distal aorta for infusion of the formalin buffered saline solution. To drain the vascular bed, an incision was made in the inferior vena cava. After perfusion fixation the vessels were carefully excised, cleaned of non-vascular tissue and stored in 4% formalin in PBS before processing for histology.

The vessel segments affected by the angioplasty procedure were cross-sectioned perpendicularly to the long axis of the arteries at 3-mm intervals. All the arterial segments were dehydrated in ethanol and xylene, embedded serially in paraffin, then sectioned (4 µm) and stained with hematoxylin-eosin and Masson's trichrome stain with Orcein.

### Assessment of Vessel Injury

All 3-mm serial segments of coronary and circumflex iliac arteries were serially examined and the site where injury was most severe and intima/neointima the greatest were identified. These regions then were used for all subsequent histological measurements to assess the effects of the different treatments on healing angioplastied arteries.

The extent of injury induced by angioplasty in the coronary arteries was classified using the gap angle of injury according to the method of Andersen et al., "Remodeling Rather Than Neointimal Formation Explains Luminal Narrowing After Deep Vessel Wall Injury: Insights From A Porcine Coronary (Re)stenosis Model", Circulation, Vol. 93, pp. 1716-1724 (1996). This method involved measuring part of the circumference of the artery in degrees, where the tunica media had been abraded and the adventitia exposed. The sides of the gap angle were drawn from the center of the lumen. The center of the lumen was defined for purposes of this experiment as the cross point of two lines drawn perpendicular to each other, with the lumen divided into four equal areas. The gap angle was measured directly with a protractor with an injury score of 360° indicating complete circumferential injury. Injury to the circumflex iliac arteries was apparent mainly as multiple small fractures in the internal elastic lamina (gap angles less than 5°) and was rarely associated with any dissection of the media.

### Morphometric Measurements/Collagen and Elastin Density

Areas of the adventitia, media, neointima and lumen, as well as vessel size were measured by projecting images of the sections onto a digitizing tablet (Complot Series 7000, Bausch and Lomb), tracing the adventitial, medial, neointimal and vessel perimeters, then calculating the relevant areas by planimetry with appropriate software such as the software described in Wong et al., "Angiotensin-Converting Enzyme Inhibition Abolishes Medial Smooth Muscle PDGF-AB Biosynthesis And Attenuates Cell Proliferation In Injured Carotid Arteries", Circulation, Vol. 96, pp. 1631-1640 (1997); and Schneider et al., "Probucol Decreases Neointimal Formation In A Swine Model Of Coronary Artery Balloon Injury", Circulation, Vol. 88, pp. 628-637 (1993). The adventitia was defined for purposes of this experiment as the area between the external elastic lamina ("EEL") and periadventitial tissues (myocardial and adipose tissue). Vessel size was defined for purposes of this experiment as the area circumscribed by the external elastic lamina. The media area was defined for purposes of this experiment as the region between the external elastic lamina and the internal elastic lamina ("IEL"). When the internal elastic lamina was missing, the area between the external elastic lamina and the remnants of medial tissue, (i.e., well-organized smooth muscle cells with intervening elastic fibers) were used instead in determining the media area. The neointima area was defined for purposes of this experiment as the region between the lumen and the internal elastic lamina. When the internal elastic lamina was missing, the area between the lumen and the remnants of medial tissue or the external elastic lamina were used instead in determining the neointima area. The lumen area was defined for purposes of this experiment as the region circumscribed by the intima/neointima-lumen border.

Collagen associated with the vessels was colored green using Masson's trichrome stain in accordance with the procedure set forth in Burke et al., "Selective Antagonism Of The ETA Receptor Reduces Neointimal Hyperplasia After Balloon-Induced Vascular Injury In Pigs", J Cardiovasc Pharmacol., Vol. 30, pp. 33-41 1997. Elastin associated with the vessels was stained black with Orcein in accordance with the procedure set forth in Lafont et al, "Endothelial Dysfunction And Collagen Accumulation. Two Independent Factors For Restenosis And Constrictive Remodeling After Experimental Angioplasty", Circulation, Vol. 100, pp. 1109-1115 (1999).

The contribution made by collagen and elastin to the different regional areas of the injured coronary arteries were then determined using a computer-interfaced color imaging system (Optimus Bioscan 2, Thomas Optical Measurement System, Inc.) to measure the fractional areas of green and black in the Masson's trichrome and Orcein stained sections, as previously described above and as futher described in Young et al., "Mineralocorticoids, Hypertension And Cardiac Fibrosis", J. Clin. Invest., Vol. 93, pp. 2578-2583 (1994). Color thresholds were applied to the acquired images so as to detect only the green (collagen)-stained and black (elastin)-stained areas.

### Statistical Analysis

Data are presented below for the coronary or circumflex iliac arteries examined in each experimental group and are expressed as means ± SEM. The significance of the differences between groups was assessed using one-way ANOVA, after testing for normality using the Kolmogorov-Smimov test (Sigmastat, Jandel Scientific) in accordance with Ward et al., "Inhibition Of Protein Tyrosine Kinase Attenuates Increases In Expression Of Transforming Growth Factor-Beta Isoforms And Their Receptors Following Arterial Injury", Arterioscler Thromb. Vasc. Biol., Vol. 17, pp. 2461-2470 (1997). Post hoc analyses utilized Newman Keuls' test. Data failing the test for "normality" were analyzed by non-parametric analysis of variance. Where differences were detected between groups, the Mann-Whitney rank sum test was used to determine their significance in accordance with Kimura et al., "Remodeling Of Human Coronary Arteries Undergoing Coronary Angioplasty Or Atherectomy", Circulation, Vol. 96, pp. 475-83 (1997).

### Injury Induced by Angioplasty in Coronary and Iliac Arteries

The doses of epleronone, spironolactone and aldosterone were well tolerated over the study period as indicated by the general well being of the animals and their intake of food.

All coronary artery segments that were collected and analyzed 28 days after angioplasty exhibited disruption of the internal elastic lumina, together with laceration of the media and exposure of the internal elastic lumina as shown in Figure 1. Table 1 reports the injury scores expressed as "gap angles" at the sites of maximal injury in coronary arteries of animals from the different treatment groups. Results are expressed as means ± SEM.

**TABLE 1**

| TREATMENT GROUP | GAP ANGLE (DEGREES) |
|---|---|
| Control (Vehicle) | 116 ± 18 |
| Spironolactone * | 111 ± 9 |
| Eplerenone | 91 ± 19 |
| Aldosterone * | 137 ± 19 |

| | |
|---|---|
| * for comparison | |

As shown in Table 1, the degree of arterial injury to the coronary arteries induced by the angioplasty procedure and assessed using gap angles ranged from 91° ± 19° to 137° ± 19° in the four groups (P > 0.05) and averaged about 114°.

Analysis of the circumflex iliac arteries also indicated that the injury level among the control, epleronone, spironolactone and aldosterone treatment groups was similar but less severe. In the circumflex iliac arteries there was little evidence of laceration of the media and exposure of the EEL as shown in Figure 1. Instead, multiple small fractures (gap angles < 5°) were observed in the internal elastic lumina.

Accordingly, the injury inflicted by the angioplasty procedure, which involved identical balloon over-sizing and pressure inflation, was generally more extensive in the coronary vessels than in the iliac vessels based on the observed extent of fracture of the internal elastic lumina and the laceration of the media. This difference in injury level consequently may have resulted in different healing responses for the two vessel types.

### Epleronone and Remodeling of Injured Coronary Arteries

The overall sizes of the angioplastied coronary arteries, as defined by the area encompassed by the external elastic lumina measured 28 days after angioplasty, were about 30% larger in the epleronone-treated animals relative to the untreated (control) animals. The lumen cross-sectional areas of the angioplastied coronary arteries were about 60% larger in the epleronone-treated animals relative to the untreated (control) animals. Only a small reduction in the developed neointima of the epleronone-treated animals was observed. Consequently, the calculated neointimal area/vessel area ratio (P <0.05 from control) was materially reduced in the eplerenone-treated animals relative to the untreated (control) animals. These experimental results showing an increase in IA/VA ratio together with an increase in lumen and overall vessel size of the healing coronary arteries one month after angioplasty indicate that epleronone attenuates constrictive remodeling of coronary arteries.

The results for the spironolactone-treated animals were qualitatively similar but less marked than observed in the eplerenone-treated animals. Higher doses of spironolactone (i.e., doses above 200 mg daily) were not tested because of the poor tolerance of the pigs to the higher doses.

The vessel cross-sectional area and the size of the lumen in aldosterone-treated animals 28 days after the angioplasty were similar to or slightly smaller than the corresponding values obtained from the untreated (control) animals. The neointima cross-sectional area and the intima area/vessel area were higher in the aldosterone-treated animals than in the untreated (control) animals, but the differences in these values were not statistical significant for the sample group. Accordingly, these results suggest that elevating circulating aldosterone concentrations does not materially affect the structure of healing of angioplastied coronary arteries.

The above results are specifically reported in Figure 2.

### Aldosterone Antagonists and Angioplastied Iliac Arteries

Treatment of the animals with either eplerenone or spironolactone at the specified doses did not appear to affect vessel area, lumen area or intima area in the injured circumflex iliac arteries relative to the untreated (control) animals based on the measurements obtained 28 days after angioplasty in the sample group tested. The size of the intima increased in the injured circumflex iliac arteries of the aldosterone-treated animals relative to the untreated (control) animals. This effect was reflected in a small reduction in vessel lumen cross sectional area and an increase in IA/VA, although these changes were not statistical significant for the sample group.

Table 2 reports the results obtained for the angioplastied circumflex iliac arteries measured 28 days after angioplasty in animals receiving spironolactone, epleronone, aldosterone or no treatment (placebo). Table 2 reports (1) vessel area as defined by the external elastic lamina, (2) lumen cross-sectional area, (3) intima area, and (4) intima area/vessel area (IA/VA). Results are expressed as means ± SEM.

**TABLE 2**

| **TREATMENT GROUP** | **VESSEL AREA (x 10⁶ µm²)** | **LUMEN AREA (x 10⁶ µm²)** | **INTIMA AREA (x 10⁶µm²)** | **1A/VA RATIO** |
|---|---|---|---|---|
| Control (Vehicle) | 1.52 ± 0.27 | 0.77 ± 0.24 | 0.13 ± 0.02 | 0.096 ± 0.021 |
| Spironolactone ** | 1.43 ± 0.14 | 0.64 ± 0.14 | 0.12 ± 0.02 | 0.089 ± 0.013 |
| Eplerenone | 1.69 ± 0.14 | 0.71 ± 0.15 | 0.15 ± 0.01 | 0.090 ± 0.010 |
| Aldosterone ** | 1.63 ± 0.13 | 0.53 ± 0.07 | 0.24 ± 0.05* | 0.144 ± 0.025 |

| | | | | |
|---|---|---|---|---|
| *P < 0.05 from control (vehicle) ** for comparison | | | | |

### Epleronone and Collagen in Angioplastied Coronary Arteries

The collagen content in the neointima and the media of the injured arteries was materially lower in eplerenone-treated animals relative to untreated (control) animals. See Figures 3 and 4A. The collagen content in each of the neointima and the media was about 65% less than the value measured for the angioplastied vessels of untreated control animals. Similarly, the collagen content in the neoadventitia was lower in the eplerenone-treated animals than in the untreated (control) animals, although this difference was smaller than observed with the neointima and the media.

A material reduction in collagen content of the neointima and media for the spironolactone-treated animals relative to the untreated (control) animals was not observed at the spironolactone dosage levels tested. See Figure 4A. A small reduction in neoadventitial collagen content, however, was observed in the spironolactone-treated animals.

The media collagen density was about 75% greater in the aldosterone-treated animals than in the untreated (control) animals. Smaller increases in collagen density (about 15%) were observed for neointima and neoadventitia in the aldosterone-treated animals relative to the untreated (control) animals.

### Aldosterone Antagonists and Elastin Content in Coronary Arteries

Elastin content was highest in the media of the angioplastied coronary arteries and lowest in the neoadventitia. See Figure 4B. The epleronone, spironolactone and the aldosterone treatments did not appear to materially affect elastin content in the different regions of these vessels in the sample groups tested.

### Summary of Results

Elevated aldosterone levels appeared to exert minimal effects on the structure of angioplastied coronary arteries although in the circumflex iliac artery an increase in neointima size was observed. In the circumflex iliac arterial bed, however, neither epleronone nor spironolactone appeared to significantly affect lumenal narrowing by preventing constrictive remodeling or neointima formation in the sample group at the dosages tested. Without being held to a particular mechanism, it is hypothesized that this differential response of the injured coronary and circumflex iliac arteries is related to the different injuries inflicted by the angioplasty in the two types of vessels with each injury evoking a different healing response. In the circumflex iliac arteries the damage involved small fractures in the internal elastic lamina and, as a consequence, neointima formation likely involves primarily the migration and proliferation of smooth muscle cells. In contrast, in the coronary arteries large fractures in the internal elastic lamina were always observed together with dissection of the media. As a consequence, smooth muscle cells and adventitial fibroblasts contribute to the neointima and changes in vessel structure within the external elastic lamina. Since the embryonic origins of smooth muscle cell in the coronary arteries are also unique, differences in their properties could also contribute to the differential responses to epleronone. In short, the study results indicate that epleronone was efficacious in attenuating collagen accumulation in these arteries at the doses tested, implicating collagen accumulation in the constrictive remodeling of angioplastied coronary arteries.

## Claims

1. The use of eplerenone for the preparation of a pharmaceutical composition for treating, inhibiting or preventing restenosis resulting from angioplasty of a blood vessel in a human subject wherein the composition contains eplerenone in an amount to administer a daily dose range of 0.5 mg to 500 mg.

2. The use of Claim 1 wherein the vessel is an artery.

3. The use of Claim 1 wherein the vessel is a coronary artery.

4. The use of Claim 1 wherein the vessel is a pulmonary artery.

5. The use of any of Claims 2, 3 or 4 wherein the composition is administered in combination with radiation therapy wherein the artery is exposed at the site of the injury to a source of radiation.

6. The use of any of Claims 1, 2, 3, 4 or 5 wherein the composition further comprises a compound selected from renin inhibitors, angiotensin I antagonists, angiotensin II antagonists, angiotensin converting enzyme inhibitors, non-steroidal diuretics, and retinoic acid, and wherein the composition is a single capsule or injection.

7. The use of any of Claims 1, 2, 3, 4 or 5 wherein the composition is coadministered with a compound selected from renin inhibitors, angiotensin I antagonists, angiotensin II antagonists, angiotensin converting enzyme inhibitors, non-steroidal diuretics, and retinoic acid.

8. A stent comprising eplerenone.

9. The stent of Claim 8 wherein the stent is an endoluminal stent.

10. The stent of Claims 8 or 9 wherein eplerenone is present on the surface of the stent.

11. The stent of Claims 8 or 9 wherein eplerenone is coated on, adsorbed on, or affixed to the stent.

12. The stent of Claims 8 or 9 wherein eplerenone is present in or on the matrix of the stent.

13. The stent of any of Claims 8 to 12 comprising eplerenone in the form of an extended release composition.

14. The stent of any of Claims 8 to 13 further comprising a second active ingredient.

15. The use of eplerenone for the preparation of a stent for treating, inhibiting or preventing restenosis of a blood vessel in a human subject.

16. The use of Claim 15 wherein the stent is an endoluminal stent.

17. The use of Claims 15 or 16 wherein eplerenone is present on the surface of the stent.

18. The use of Claims 15 or 16 wherein eplerenone is present in or on the matrix of the stent.

19. The use of Claims 15 or 16 wherein eplerenone is coated on, adsorbed on, or affixed to the stent.

20. The use of any of Claims 15 to 19 wherein the stent comprises eplerenone in the form of an extended release composition.

21. The use of any of Claims 15 to 20 wherein the stent further comprises a second active ingredient.

## Patentansprüche

1. Verwendung von Eplerenon für die Herstellung einer pharmazeutischen Zusammensetzung zur Behandlung, Inhibierung oder Prävention von Restenose, die aus einer Angioplastie eines Blutgefäßes bei einem menschlichen Subjekt resultiert, wobei die Zusammensetzung Eplerenon in einer Menge, um eine tägliche Dosis im Bereich von 0,5 mg bis 500 mg zu verabreichen, enthält.

2. Verwendung nach Anspruch 1, wobei das Gefäß eine Arterie ist.

3. Verwendung nach Anspruch 1, wobei das Gefäß eine Koronararterie ist.

4. Verwendung nach Anspruch 1, wobei das Gefäß eine Lungenarterie ist.

5. Verwendung nach einem der Ansprüche 2, 3 und 4, wobei die Zusammensetzung in Kombination mit Strahlungstherapie verabreicht wird, wobei die Arterie an der Stelle der Schädigung einer Strahlungsquelle ausgesetzt wird.

6. Verwendung nach einem der Ansprüche 1, 2, 3, 4 und 5, wobei die Zusammensetzung außerdem eine Verbindung umfasst, ausgewählt aus Renininhibitoren, Angiotensin-I-Antagonisten, Angiotensin-II-Antagonisten, Inhibitoren des Angiotensin-umwandelnden Enzyms, nicht-steroidalen Diuretika und Retinoesäure, umfasst und wobei die Zusammensetzung eine einzelne Kapsel oder Injektion ist.

7. Verwendung nach einem der Ansprüche 1, 2, 3, 4 und 5, wobei die Zusammensetzung mit einer Verbindung, ausgewählt aus Renininhibitoren, Angiotensin-I-Antagonisten, Angiotensin-II-Antagonisten, Inhibitoren des Angiotensin-umwandelnden Enzyms, nicht-steroidalen Diuretika und Retinoesäure, coverabreicht wird.

8. Stent, umfassend Eplerenon.

9. Stent nach Anspruch 9, wobei der Stent ein endoluminaler Stent ist.

10. Stent nach Anspruch 8 oder 9, wobei Eplerenon an der Oberfläche des Stents vorliegt.

11. Stent nach Anspruch 8 oder 9, wobei Eplerenon auf den Stent aufgetragen, an dem Stent adsorbiert oder an dem Stent fixiert ist.

12. Stent nach Anspruch 8 oder 9, wobei Eplerenon in oder an der Matrix des Stents vorliegt.

13. Stent nach einem der Ansprüche 8 bis 12, umfassend Eplerenon in Form einer Extended-Release-Zusammensetzung.

14. Stent nach einem der Ansprüche 8 bis 13, der außerdem ein zweites aktives Ingredienz umfasst.

15. Verwendung von Eplerenon für die Herstellung eines Stents zur Behandlung, Inhibierung oder Prävention von Restenose eines Blutgefäßes bei einem menschlichen Subjekt.

16. Verwendung nach Anspruch 15, wobei der Stent ein endoluminaler Stent ist.

17. Verwendung nach Anspruch 15 oder 16, wobei Eplerenon an der Oberfläche des Stents vorliegt.

18. Verwendung nach Anspruch 15 oder 16, wobei Eplerenon in oder an der Matrix des Stents vorliegt.

19. Verwendung nach Anspruch 15 oder 16, wobei Eplerenon auf den Stent aufgetragen, an dem Stent adsorbiert oder an dem Stent fixiert ist.

20. Verwendung nach einem der Ansprüche 15 bis 19, wobei der Stent Eplerenon in Form einer Extended-Release-Zusammensetzung umfasst.

21. Verwendung nach einem der Ansprüche 15 bis 20, wobei der Stent außerdem ein zweites aktives Ingredienz umfasst.

## Revendications

1. Utilisation d'éplérénone pour la préparation d'une composition pharmaceutique destinée au traitement, à l'inhibition ou à la prévention de la resténose résultant d'une angioplastie d'un vaisseau sanguin chez un sujet humain dans laquelle la composition contient de l'éplérénone en quantité suffisante pour administrer une dose journalière dans la plage de 0,5 mg à 500 mg.

2. Utilisation selon la revendication 1, dans laquelle le vaisseau est une artère.

3. Utilisation selon la revendication 1, dans laquelle le vaisseau est une artère coronaire.

4. Utilisation selon la revendication 1, dans laquelle le vaisseau est une artère pulmonaire.

5. Utilisation selon l'une quelconque des revendications 2, 3 ou 4, dans laquelle la composition est administrée en association avec la radiothérapie, dans laquelle l'artère est exposée au site de la blessure à une source de radiation.

6. Utilisation selon l'une quelconque des revendications 1, 2, 3, 4 ou 5, dans laquelle la composition comprend en outre un composé choisi parmi les inhibiteurs de rénine, les antagonistes de l'angiotensine I, les antagonistes de l'angiotensine II, les inhibiteurs de l'enzyme de conversion de l'angiotensine, les diurétiques non stéroïdiens, et l'acide rétinoïque, et dans laquelle la composition est une capsule ou une injection unique.

7. Utilisation selon l'une quelconque des revendications 1, 2, 3, 4 ou 5 dans laquelle la composition est co-administrée avec un composé choisi parmi les inhibiteurs de rénine, les antagonistes de l'angiotensine I, les antagonistes de l'angiotensine II, les inhibiteurs de l'enzyme de conversion de l'angiotensine, les diurétiques non stéroïdiens, et l'acide rétinoïque.

8. Endoprothèse vasculaire comprenant de l'éplérénone.

9. Endoprothèse vasculaire selon la revendication 8 dans laquelle l'endoprothèse est une endoprothèse endoluminale.

10. Endoprothèse vasculaire selon la revendication 8 ou 9, dans laquelle l'éplérénone est présente à la surface de l'endoprothèse.

11. Endoprothèse vasculaire selon la revendication 8 ou 9, dans laquelle l'éplérénone est enduite sur, adsorbée sur ou fixée à l'endoprothèse.

12. Endoprothèse vasculaire selon la revendication 8 ou 9, dans laquelle l'éplérénone est présente dans ou sur la matrice de l'endoprothèse.

13. Endoprothèse vasculaire selon l'une quelconque des revendications 8 à 12, qui comprend de l'éplérénone sous forme d'une composition à libération prolongée.

14. Endoprothèse vasculaire selon l'une quelconque des revendications 8 à 13, qui comprend en outre un second principe actif.

15. Utilisation d'éplérénone pour la préparation d'une endoprothèse vasculaire destinée au traitement, à l'inhibition ou la prévention de la resténose d'un vaisseau sanguin chez un sujet humain.

16. Utilisation selon la revendication 15, dans laquelle l'endoprothèse vasculaire est une endoprothèse endoluminale.

17. Utilisation selon la revendication 15 ou 16, dans laquelle l'éplérénone est présente à la surface de l'endoprothèse.

18. Utilisation selon la revendication 15 ou 16, dans laquelle l'éplérénone est présente dans ou sur la matrice de l'endoprothèse.

19. Utilisation selon la revendication 15 ou 16, dans laquelle l'éplérénone est enduite sur, adsorbée sur ou fixée à l'endoprothèse.

20. Utilisation selon l'une quelconque des revendications 15 à 19, dans laquelle l'endoprothèse comprend de l'éplérénone sous forme d'une composition à libération prolongée.

21. Utilisation selon l'une quelconque des revendications 15 à 20, dans laquelle l'endoprothèse comprend en outre un second principe actif.
